# FASCICULE DE BREVET EUROPEEN

(11) **EP 3 934 731 B1**
(45) Date de publication et mention de la délivrance du brevet: **04.06.2025**
(21) Numéro de dépôt: 20708493.0
(22) Date de dépôt: 04.03.2020
(51) Int. Cl.: A61M 35/00, A61F 9/00

(54) **DISPOSITIF DE DISTRIBUTION D'UNE SOLUTION**
VORRICHTUNG ZUR ABGABE EINER LÖSUNG
DEVICE FOR DISPENSING A SOLUTION

(30) Priorité: 05.03.2019 FR 1902248
(43) Date de publication de la demande: 12.01.2022
(73) Titulaire: NEMERA LA VERPILLIERE, 38290 La Verpillière (FR)
(72) Inventeur: DECOCK, Thierry, 69007 Lyon (FR); CERQUEIRA, Guillaume, 69007 Lyon (FR); PAINCHAUD, Gaëtan, 69340 Francheville (FR)
(74) Mandataire: LLR
(86) Numéro de dépôt international: PCT/EP2020/055753
(87) Numéro de publication internationale: WO 2020/178358

(56) Documents cités:
- CN-A- 107 096 122
- CN-A- 109 331 332
- CN-U- 204 134 034
- DE-A1- 102011 009 504
- US-A1- 2005 201 811
- US-A1- 2018 177 957
- US-B1- 9 072 581

## Description

L'invention concerne un dispositif de distribution d'une solution à délivrer sur une partie corporelle d'un patient. Il s'agit notamment d'un produit liquide à distribuer dans un organe, tel que par exemple un œil ou une zone de la peau, que l'on appelle zone de distribution. Le patient peut être un humain ou encore un animal.

On connait déjà, dans l'état de la technique, des dispositifs de distribution de produits liquides, tels que celui décrit dans le document US9072581B1. Le document JP2018094420 décrit un dispositif d'assistance à l'utilisation d'un dispositif de distribution d'une solution ophtalmique, comprenant une partie de préhension, comprenant des zones de préhension concaves pour un serrage entre des doigts de l'utilisateur, permettant une activation de la distribution de solution ophtalmique. Le dispositif comporte en outre une partie d'appui sur le visage de l'utilisateur, autour de l'œil. Ce dispositif a vocation à rendre plus confortable la distribution de solution ophtalmique à un utilisateur du dispositif. La situation peut toutefois être améliorée.

L'invention a notamment pour but de fournir un dispositif de distribution d'une solution, permettant une distribution plus confortable pour l'utilisateur.

L'invention propose à cet effet un dispositif de distribution d'une solution comme dans la revendication 1 comprenant :
- un corps dont la surface extérieure est destinée à être tenue par au moins une main d'un utilisateur et comprend au moins une zone d'activation apte à être sollicitée par l'utilisateur pour provoquer une distribution de la solution, et,
- une tête portée par le corps, la tête étant pourvue d'une surface d'appui sur une zone du corps d'un patient, formant un contour extérieur, et d'au moins un orifice de distribution de la solution,
le dispositif de distribution étant agencé de sorte que la tête est couplée au corps par des moyens de liaison autorisant sa rotation par rapport au corps autour d'un axe de rotation sensiblement parallèle à un axe du corps afin de modifier la position de la surface d'appui par rapport à la au moins une zone d'activation.

Grâce à la liaison autorisant la rotation de la tête par rapport au corps, il est possible de modifier l'orientation de la tête, par rapport au corps du dispositif de distribution utilisé pour sa préhension, et donc de la surface d'appui par rapport à la zone d'activation située sur le corps. De ce fait, lorsque la tête doit prendre une position spécifique, par exemple dans le cas où elle présente une forme complémentaire à celle d'un contour d'un œil du patient pour pouvoir être correctement calée, le corps peut être orienté par rapport à la tête pour faciliter la préhension par l'utilisateur et/ou l'activation par l'utilisateur de la distribution de la solution. Par exemple, l'utilisateur peut utiliser indifféremment sa main gauche ou sa main droite pour manipuler le corps et/ou activer la distribution de la solution. Il en résulte que l'on peut améliorer l'ergonomie d'un dispositif de distribution d'une solution sur une zone du corps d'un patient, apporter un plus grand confort pour l'utilisateur et une meilleure précision pour la distribution de la solution, ou encore mieux s'adapter à la morphologie propre à chaque patient et/ou utilisateur du dispositif de distribution. L'utilisateur tenant le corps du dispositif de distribution peut être le patient à qui doit être distribuée la solution ou encore un utilisateur tiers qui assiste ce patient, tel qu'un membre du personnel médical.

Un autre avantage est que l'utilisateur peut orienter le corps une fois que la tête est positionnée sur la zone du corps du patient, évitant donc un déplacement inopiné de la surface d'appui par rapport à la zone du corps du patient (et plus précisément à la zone de distribution) au moment de la distribution.

Cette possibilité d'orienter le corps par rapport à la tête, et donc par rapport à la surface d'appui, est particulièrement intéressante lorsque la au moins une zone d'activation présente une surface restreinte, disposée selon un secteur angulaire seulement de la surface périphérique (c'est-à-dire lorsque la au moins une zone d'activation a une forme distincte d'une forme de révolution, ce qui impose un positionnement des doigts de l'utilisateur prédéterminé sur le corps). On entend par « zone d'activation disposée sur la surface périphérique et délimitant un secteur angulaire » une zone d'activation qui n'est pas disposée continûment sur un contour de la zone périphérique. En d'autres termes, la surface périphérique comprend au moins un contour comprenant la au moins une zone d'activation et une zone dite inerte, juxtaposée à la au moins une zone d'activation. Ainsi, la au moins zone d'activation a une forme distincte d'une zone de révolution, ce qui rend le dispositif de distribution proposé d'autant plus ergonomique et intuitif pour l'activation de la distribution de la solution par l'utilisateur.

Suivant d'autres caractéristiques optionnelles du dispositif de distribution prises seules ou en combinaison :
- La tête du dispositif de distribution a un contour dont la forme est distincte d'une forme de révolution, par exemple un contour de section ovale ou oblongue. Le terme « forme de révolution » désigne une forme invariante par rotation autour d'un axe fixe dit « axe de révolution » et dont toute intersection avec un plan perpendiculaire à l'axe de révolution définit un cercle. Ceci permet une meilleure adaptabilité à la physiologie du patient et plus de confort pour l'utilisation du dispositif de distribution, tout particulièrement si la zone de distribution se situe autour d'un œil du patient.
- Le corps a un contour dont la forme est distincte d'une forme de révolution, par exemple un contour de section ovale. Dans ce cas, il est particulièrement intéressant de pouvoir modifier l'orientation du corps par rapport à la tête car l'utilisateur saisit le corps en posant ses doigts de part et d'autre du diamètre long de la partie ovale (pour faciliter l'appui), et il est plus confortable que l'orientation de ce diamètre long du corps ne soit pas imposée par la position de la tête.
- Les moyens de liaison sont aptes à opérer une liaison d'un type choisi dans le groupe comprenant au moins : une liaison pivot, une liaison rotule, une liaison pivot glissant. Avantageusement, les moyens de liaison sont configurés pour assurer à la fois un guidage en rotation selon l'axe du corps du dispositif de distribution et une fixation dans la direction axiale du corps du dispositif de distribution par rapport à sa tête. Par ailleurs, un élément de guidage comprend par exemple une nervure annulaire ou semi-annulaire, ou encore un ou plusieurs pions de guidage. De préférence, la rotation établie entre le corps et la tête comprend une liaison pivot selon un axe d'orientation sensiblement parallèle à l'axe du corps. Par ailleurs, l'axe du corps correspond de préférence à un axe central du corps.
- Les moyens de liaison comprennent des éléments de butée aptes à limiter la rotation relative du corps du dispositif de distribution et de sa tête. Il est ainsi possible de limiter le débattement angulaire du corps et de la tête entre eux. Ceci peut permettre par exemple de limiter des risques de fragilisation du dispositif de distribution par arrachage, en évitant que la tête ne présente une trop grande surface dépassant du corps selon l'orientation et/ou la rotation choisie par l'utilisateur. De préférence, les éléments de butée sont configurés pour permettre un angle de débattement maximal compris entre 0° et 90°, de préférence encore entre 0° et 45°.
- La tête et le corps peuvent prendre une position relative dite de stockage et les moyens de liaison comprennent des moyens de rappel de la tête en position de stockage. Dans cette position de stockage les contours des corps et de la tête peuvent être dans l'alignement l'un de l'autre lorsqu'ils sont similaires. Ceci apporte de la compacité et limite conséquemment les risques d'arrachage de la tête au moment du conditionnement, notamment pour le stockage.
- La tête est montée détachable par rapport au corps. Cela permet l'adaptation de différentes têtes interchangeables sur le corps pour augmenter la durée de vie du dispositif de distribution, ou encore pour mieux s'adapter à la morphologie d'un patient selon la zone du corps du patient où on veut distribuer la solution via le dispositif de distribution.
- Les moyens de liaison comprennent des moyens de définition d'au moins une position prédéterminée, par exemple un cran de positionnement, ou encore des moyens de friction.
- La tête comprend des moyens de détection de solution distribuée, de préférence optoélectroniques.
- Le dispositif de distribution comprend une interface de connexion électrique entre le corps et la tête, comprenant au moins un élément conducteur, de préférence un élément annulaire conducteur coopérant par contact physique avec un élément complémentaire conducteur.
- Le corps est configuré pour recevoir deux parties d'appui de forme distincte. Par exemple et de façon avantageuse, la tête est réalisée dans un matériau thermo-formable, le corps dans un autre matériau.

L'invention sera mieux comprise à la lecture de la description qui va suivre donnée uniquement à titre d'exemple et faite en se référant aux dessins annexés dans lesquels :
[Fig. 1] La figure 1 est une vue en perspective d'un dispositif de distribution selon un premier mode de réalisation de l'invention
[Fig. 2] La figure 2 est une vue éclatée et en perspective d'une partie du dispositif de distribution représenté sur la figure 1.
[Fig. 3] La figure 3 comprend des vues de faces et de haut du dispositif de distribution déjà représenté sur les figures 1 et 2, illustrant différentes positions de la tête du dispositif de distribution par rapport au corps du dispositif de distribution.
[Fig. 4] La figure 4 comprend une vue éclatée et en perspective d'un dispositif de distribution selon un second mode de réalisation de l'invention ainsi, qu'une vue après assemblage de la tête du dispositif de distribution sur le corps et orientation de la tête du dispositif de distribution par rapport au corps.
[Fig. 5] La figure 5 est une vue éclatée et en perspective d'un dispositif de distribution selon un troisième mode de réalisation de l'invention.

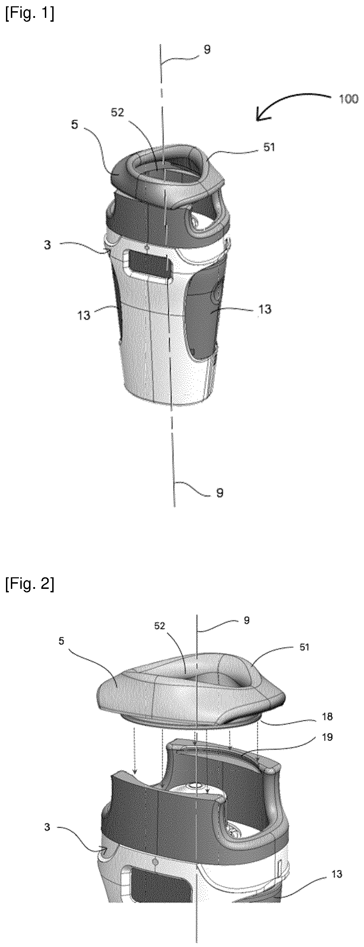

La figure 1 illustre un dispositif de distribution 100 selon un premier mode particulier et non limitatif de l'invention. Le dispositif de distribution 100 comprend un corps 3 et une tête 5. Le dispositif de distribution 100 présente une forme telle qu'une section de son corps 3 est de forme globalement ovale ou oblongue, c'est-à-dire que le corps a une forme distincte d'une forme de révolution. La tête 5 présente elle aussi une forme globale présentant une section ovale ou oblongue, ce qui revient à dire que la tête 5 présente une forme distincte d'une forme de révolution. La surface extérieure 7 du corps 3 du dispositif de distribution 100 est destinée à être tenue par au moins une main d'un utilisateur pour la distribution d'une solution contenue dans le dispositif de distribution 100. L'utilisateur tenant le corps 3 du dispositif de distribution 100 peut être le patient à qui doit être distribuée la solution ou encore un utilisateur tiers qui assiste ce patient, tel qu'un membre du personnel médical.

De façon optionnelle, le corps 3 comprend des moyens d'affichage 11 utiles à un bon suivi de la distribution de la solution contenue dans le dispositif de distribution 100.

La surface extérieure 7 du corps 3 du dispositif de distribution 100 comprend au moins une zone d'activation 13 apte à être sollicitée par l'utilisateur pour provoquer une distribution de la solution contenue dans le dispositif de distribution 100. Selon un mode de réalisation préféré de l'invention, deux zones d'activation 13 comprennent chacune un élément mécanique configuré pour comprimer un réservoir intérieur au corps 3 du dispositif de distribution 100 en vue de distribuer une solution contenue dans le réservoir. Ces éléments mécaniques sont rappelés dans une position correspondant à une absence de distribution par des moyens de rappel. Les moyens de rappel peuvent être apportés par une élasticité du réservoir, ou par des éléments ajoutés tels que des ressorts.

La tête 5 du dispositif de distribution 100 est portée par le corps 3. La tête 5 est pourvue d'une surface d'appui 51 sur une zone du corps d'un patient recevant la solution à distribuer. La tête 5 comprend également un orifice 52 (ou ouverture) de distribution de la solution vers une zone du corps du patient à qui la solution est distribuée. Selon la configuration de la tête 5, la surface d'appui 51 peut former un contour de l'orifice 52, par exemple lorsque le dispositif de distribution 100 est configuré pour la délivrance d'un produit ophtalmique. Avantageusement, le contour peut être d'une forme distincte d'une forme de révolution, telle que par exemple une forme ovale, de sorte à optimiser le confort d'une distribution d'une solution ophtalmique sous forme de gouttes. Selon d'autres variantes, la surface d'appui peut être plane et comprendre un ou plusieurs orifices, pour une distribution sur une zone de l'épiderme d'un patient. Selon une autre variante, la tête peut prendre la forme d'un embout dont la forme est adaptée pour une application nasale ou une distribution par voie orale. La tête peut aussi comprendre une forme concave délimitée par un contour en saillie, la partie concave étant pourvue d'un ou plusieurs orifices pour délivrer la solution contenue dans le dispositif de distribution 100 sur une zone du corps du patient délimitée par l'application du contour saillant sur l'épiderme. Ces exemples ne sont bien évidemment pas limitatifs.

Avantageusement, la tête 5 du dispositif de distribution 100 est couplée au corps 3 par des moyens de liaison autorisant sa rotation par rapport au corps 3 afin de modifier la position de la surface d'appui 51 par rapport à la au moins une zone d'activation 13.

Selon le mode de réalisation préféré de l'invention représenté sur la figure 1, la tête 5 est couplée au corps 3 du dispositif de distribution 100 par une liaison pivot permettant une rotation de la tête 5 autour d'un axe de rotation 9 sensiblement parallèle à l'axe du corps 3. Par ailleurs, l'axe du corps 3 correspond de préférence à un axe central du corps 3.

Selon des exemples, les moyens de liaison sont configurés pour permettre une liaison pivot glissant de la tête 5 par rapport au corps 3 ou forment une liaison rotule entre le corps 3 et la tête 5.

Le dispositif de distribution 100 est configuré pour pouvoir contenir et distribuer une solution se présentant aussi bien sous forme liquide que sous forme d'un gel ou d'une mousse.

Le dispositif de distribution 100 est en outre apte à délivrer la solution, en fonction de sa forme, pour une application directe sur une zone du corps du patient, ou encore en spray ou sous forme de brouillard lorsque la solution est liquide.

Ainsi, le dispositif de distribution 100 peut être adapté à des applications variées telles que la délivrance de médicament ou de produits paramédicaux, la délivrance de produits de nettoyage tels que des collyres, par exemple, ou encore dans le domaine vétérinaire, ces exemples n'étant pas limitatifs.

La rotation de la tête 5 du dispositif de distribution 100 par rapport à son corps 3 est tout particulièrement intéressante lorsque la zone d'activation 13 présente une surface restreinte ou à tout le moins disposée selon un secteur angulaire seulement de la surface extérieure du corps 3. En d'autres termes, cela revient à dire que la zone d'activation 13 a une forme distincte de la forme d'une révolution et que l'utilisateur ne peut saisir le corps 3 selon n'importe quelle position de sa main ou de ses doigts quand la zone d'activation 13 apparait de taille restreinte par rapport à la surface totale extérieure 7 du corps ou si l'activation de la distribution requiert une intervention sur plusieurs zones d'activation 13 simultanément. C'est le cas, par exemple, si le corps 3 dispose de deux zones d'activation 13 disposées de part et d'autre et de façon diamétralement opposées par rapport à l'axe de rotation 9, tel que représenté sur la figure 1.

La figure 1 représente un corps 3 comprenant deux zones d'activation 13, chacune ayant une forme distincte d'une forme de révolution. L'utilisateur du dispositif de distribution 100 doit donc saisir le corps 3 selon une position prédéterminée de ses doigts, en particulier quand les zones d'activation 13 sont de taille restreinte par rapport à la surface totale extérieure du corps 3.

Selon le mode de réalisation représenté sur la figure 1, les moyens de liaison entre le corps 3 et la tête 5 sont adaptés pour qu'une rotation de la tête 5 par rapport au corps 3 soit assurée par emboitement de la tête 5 sur une première surface annulaire du corps 3 comprenant au moins une nervure 19 (visible sur la figure 2). Ainsi une rainure 18 (visible sur la figure 2) agencée sur une seconde surface annulaire de la tête 5 est combinée avec la nervure 19 agencée sur la première surface annulaire du corps 3, après assemblage. De façon similaire la rainure 18 peut être agencée sur le corps 3 et la nervure 19 être alors agencée sur la tête 5. Selon une variante, plusieurs rainures et nervures sont combinées pour constituer les moyens de liaison entre le corps 3 et la tête 5. Selon ce mode de réalisation, la liaison ainsi établie permet un blocage en translation de la tête 5 par rapport au corps 3 mais autorise une rotation complète de la tête 5 autour de l'axe de rotation 9.

Astucieusement, les surfaces annulaires du corps 3 prennent la forme d'arcs de cercle diamétralement opposés par rapport à l'axe de rotation 9 de sorte qu'un couplage entre la tête 5 et le corps 3 puisse être réalisé sans avoir à développer une force trop conséquente, en bénéficiant éventuellement de l'élasticité des matériaux utilisés pour la fabrication du corps 3 et/ou de la tête 5 dans la zone de liaison. La liaison entre la tête 5 et le corps 3 est ainsi réalisée par « clipsage » (ou encliquetage).

Ainsi, la tête 5 peut être avantageusement montée de façon détachable par rapport au corps 3, ce qui procure l'avantage de pouvoir remplacer la tête 5 et prolonger la durée d'utilisation du dispositif de distribution 100 ou encore pouvoir utiliser une pluralité de têtes, par exemple chacune adaptée à une utilisation particulière ou à une morphologie du patient a qui la solution doit être distribuée.

Il est par exemple possible de choisir la tête 5 en fonction de la dimension d'un élément propre à la physionomie du patient, en fonction de sa taille ou de sa forme ou de sa fragilité. Ces exemples n'étant pas limitatifs.

Il est par exemple possible d'utiliser une tête 5 dont le contour présente une surface d'appui 51 de dimensions réduites pour une délivrance d'un produit ophtalmique ou un produit de nettoyage à un nourrisson ou un enfant en bas âge, alors qu'une tête présentant un contour plus grand sera préférée pour un même type de délivrance à un adulte.

La figure 2 est une vue éclatée et en perspective de la partie supérieure du dispositif de distribution 100 représenté sur la figure 1 illustrant les moyens de liaison 18, 19 permettant d'assurer une liaison en rotation de la tête 5 par rapport au corps 3, autour de l'axe de rotation 9 du dispositif de distribution 100.

En fonction de la configuration du dispositif de distribution 100, l'axe de rotation 9 peut correspondre à un axe de distribution de la solution contenue dans le dispositif de distribution 100. Il est entendu par « axe de distribution » un axe défini par la direction de déplacement imposée à la solution lors d'une distribution à travers le ou les orifices de distribution agencés dans le corps 3.

Avantageusement, les moyens de liaison 18, 19 comprennent des moyens de définition d'au moins une position prédéterminée, par exemple un cran de positionnement, ou encore des moyens de friction. Il est ainsi possible de limiter la rotation la tête 5 par rapport au corps 3 de de sorte que des parties de la tête 5 ne soient pas trop saillantes par rapport à l'ensemble du dispositif de distribution 100 et/ou que cette configuration ne présente un risque d'arrachage accidentel de la tête 5. C'est particulièrement le cas lorsque l'ensemble du dispositif de distribution 100 présente une section de forme globale ovale ou oblongue, permettant notamment une meilleure préhension ou une ergonomie adaptée à une activation via les deux zones d'activation 13, par l'utilisation par exemple, de deux doigts de l'utilisateur.

Selon un mode de réalisation de l'invention, le dispositif de distribution 100 comprend des moyens de rappel de la tête 5 dans une position de stockage par rapport au corps 3. Ainsi, si la tête 5 est ramenée en position de stockage et l'ensemble du dispositif de distribution 100 présente dans cette position de stockage un profil adapté à son introduction dans un élément de conditionnement tel qu'une boite ou qu'un étui, le rangement du dispositif de distribution 100 dans l'élément de conditionnement s'en trouve facilité.

Les moyens de rappel peuvent comprendre deux points de fixation d'un ressort utilisé en traction ou en compression avec, couplés au ressort, un premier point de fixation étant disposé sur le corps 3 et un second point de fixation sur la tête 5. Selon une variante, l'effet ressort peut être produit par combinaison d'un ergot se déplaçant le long d'une languette souple présentant une épaisseur croissant au fil du déplacement de la tête 5 par rapport au corps 3, de sorte que la languette souple opère comme un ressort en compression. Selon cette configuration la languette peut être fixée solidairement du corps 3 et l'ergot exerçant un appui sur la languette fixé solidairement de la tête 5, ou inversement. Ces exemples n'étant pas limitatifs.

Selon un mode de réalisation exemplaire, les moyens de liaison de la tête 5 sur le corps 3 comprennent au moins un élément de guidage en translation de la tête 5 par rapport au corps 3, outre la capacité à offrir une liaison pivot. Par exemple, un tronc supportant des éléments de liaison tels que précités du corps 3 porte ces éléments de liaison montés sur un cylindre glissant dans un tube agencé dans le corps au moyen d'une liaison étanche, le tronc étant monté sur un ressort en compression de sorte à créer un effet d'amortissement lors de l'établissement d'un contact entre la surface d'appui 51 de la tête 5 et la zone du corps d'un patient devant recevoir la solution. Ainsi, le contact avec des zones fragiles du corps du patient est amélioré et le confort d'utilisation en est accru. Ceci est particulièrement avantageux pour une distribution d'une solution ophtalmique dans un œil du patient.

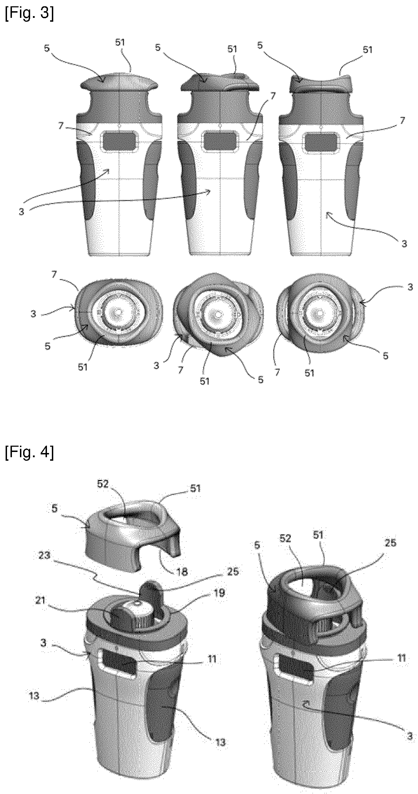

La figure 3 illustre différentes positions de la tête 5 du dispositif de distribution 100 par rapport à son corps 3. La partie supérieure de la figure comprend trois vues de face du dispositif de distribution 100 correspondant respectivement à trois positions relatives de la tête 5 par rapport au corps 3 du dispositif de distribution 100. La partie inférieure de la figure comprend trois vues de haut du dispositif de distribution 100 en correspondance des vues de faces représentées dans la partie haute.

Ainsi, les vues de face et de haut positionnées à gauche sur la figure 3 représentent le dispositif de distribution 100 lorsque la tête 5 et orientée dans une position nominale, ou encore dite de position de stockage. Dans cette position de stockage, la tête 5 présente un angle de rotation nul par rapport au corps 3.

Les figures de face et de haut, positionnées au centre sur la figure 3, représentent le dispositif de distribution 100 de distribution lorsque la tête 5 a été orientée par une rotation selon un angle de 45 degrés dans le sens horaire par rapport au corps 3, et au regard de la position de stockage représentée à gauche sur la même figure. Ceci permet avantageusement d'offrir une meilleure ergonomie et un plus grand confort d'utilisation lorsque l'utilisateur applique la surface d'appui 51 de la tête 5 sur une zone du corps d'un patient, telle que le contour d'un œil par exemple, et active immédiatement après une distribution en intervenant manuellement sur la ou les zones d'activation 13, en particulier lorsque la zone d'activation 13 est définie selon un secteur angulaire sur la surface extérieure 7 du corps 3.

Les figures de face et de haut, positionnées à droite sur la figure 3, représentent le dispositif de distribution 100 lorsque la tête 5 a été orientée par une rotation selon un angle de 90 degrés dans le sens horaire ou antihoraire par rapport au corps 3 est au regard de la position de stockage représentée à gauche sur la même figure.

Il est possible de constater que, selon les configurations de formes en correspondance du mode de réalisation représenté, une orientation de la tête 5 à 45 degrés ou à 90 degrés par rapport à la position de stockage est de nature à présenter des surfaces s'étendant plus largement que la surface extérieure 7 du corps 3, selon certaines directions, présentant alors des risques d'accrochage ou d'arrachement accidentel de la tête 5. Cela illustre l'intérêt d'inclure des moyens de rappel de la tête 5 dans la position nominale représentée à gauche et utilisée pour le stockage du dispositif de distribution 100 dans un élément de conditionnement tel qu'une boite ou un étui adapté à la forme globale du dispositif de distribution 100.

La figure 4 représente une vue éclatée (partie gauche de la figure) et une vue non-éclatée (partie droite de la figure), en perspective, du dispositif de distribution 100 selon un second mode de réalisation de l'invention.

Selon ce mode de réalisation de l'invention les moyens de liaison 18, 19 sont agencés sur une surface plane ou sensiblement plane du corps 3 et la tête 5 occupe un volume plus conséquent formant une coiffe adaptée à cacher et protéger des languettes 21 et 23 portant des moyens de détection de la distribution de la solution contenue dans le dispositif de distribution 100.

Avantageusement le dispositif de distribution 100 comprend des moyens de mesure de la distribution et de la quantité de solution distribuée, ce qui permet de définir très précisément la quantité de solution délivrée à un patient. Cette fonction du dispositif de distribution 100 est particulièrement appréciable pour la délivrance de produits pour laquelle une efficacité maximale correspond à une dose comprise dans un intervalle de valeurs de volume de solution prédéterminé.

Les moyens d'affichage 11 permettent de délivrer des informations en ce sens à un utilisateur ou à un tiers susceptible d'opérer un suivi en lien avec une distribution de solution à l'aide du dispositif de distribution 100.

Selon un mode de réalisation préféré, les moyens de détection de la solution distribuée sont de type optoélectroniques. Par exemple, le corps 3 comprend les languettes 21 et 23 supportant partiellement les moyens de liaison 18, 19 entre le corps 3 et la tête 5 et les languettes 21 et 23 sont agencées face-à-face et de façon diamétralement opposés par rapport à l'axe de rotation 9 de distribution de la solution. L'une des languettes 21, 23 comprend un émetteur infra-rouge 24, non visible sur la figure, et l'autre languette 23, 21 comprend un récepteur infra-rouge 25 configuré pour détecter le rayonnement émis par l'émetteur infra-rouge disposé en regard. Ainsi lorsqu'une distribution de solution est opérée, la solution distribuée interrompt le rayonnement infra-rouge entre l'émetteur infra-rouge 24 et le récepteur infra-rouge 25 et il est possible d'évaluer la quantité de solution distribuée en fonction du temps cumulé d'interruption du rayonnement pendant l'activation par l'utilisateur du dispositif de distribution 100. Bien évidemment une telle configuration requiert l'usage d'une unité de contrôle apte à piloter les éléments optoélectroniques ainsi que des éléments de restitution visuels ou sonore pour délivrer des informations en lien avec la distribution. Une telle unité de contrôle comprend classiquement un microcontrôleur alimenté sur batterie rechargeable ou remplaçable, ainsi que les différents éléments usuellement utilisés pour implémenter une telle unité de contrôle embarquée dans un dispositif de distribution 100 portable, ou un système électronique équivalent. Selon une telle configuration, l'ensemble des moyens de mesure de la distribution est intégré au corps 3 et il n'est pas utile de prévoir à cet effet des moyens de connexion entre le corps 3 et la tête 5.

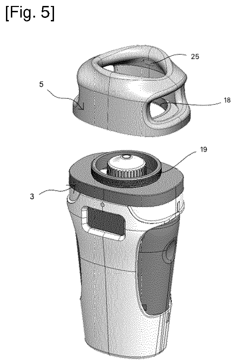

La figure 5 représente une vue éclatée et en perspective du dispositif de distribution 100 selon un troisième mode de réalisation de l'invention. Selon ce mode de réalisation, la tête 5 présente une forme similaire à celle décrite dans le second mode de réalisation décrit ci-avant et illustré par la figure 4, mais comprend également les moyens optoélectroniques de détection de la solution lors d'une opération de distribution. Ainsi, la présence de languettes 21, 23 agencées sur la surface supérieure plane du dispositif de distribution 100 n'est plus nécessaire. Selon cette configuration les moyens de liaison 18, 19 que sont les surfaces annulaires complémentaires, comprenant respectivement au moins une nervure 19 et une rainure 18, comprennent des éléments conducteurs pour assurer autant de connexions électriques qu'il est nécessaire d'établir pour la mise en œuvre des moyens optoélectroniques de mesure. Les éléments conducteurs sont par exemple des couronnes et anneaux combinés de sorte à opérer des liaisons mécaniques et donc électriques entre les moyens optoélectroniques et l'unité de contrôle embarquée, y compris lorsque la tête 5 est mue en rotation par rapport au corps 3. Selon une variante du mode de réalisation, la surface plane ou sensiblement plane supérieure du corps 3 est équipée de frotteurs réalisés dans un matériau conducteur et connectés à l'unité de contrôle, et la tête 5 est équipée de couronnes elles aussi réalisées dans un matériau conducteur et connectées aux moyens optoélectroniques. Selon un mode de réalisation de l'invention, quatre couronnes logées dans la tête 5 sont respectivement connectées à quatre frotteurs solidaires du corps 3 pour connecter l'émetteur infra-rouge 24 et le récepteur infra-rouge 25 à l'unité de contrôle embarquée dans le corps 3 du dispositif de distribution 100.

En d'autres termes, et selon un mode de réalisation de l'invention, le dispositif de distribution 100 d'une solution comprend le corps 3 dont la surface extérieure 7 est destinée à être tenue par au moins une main d'un utilisateur et comprenant au moins une zone d'activation 13 apte à être sollicitée par l'utilisateur pour provoquer une distribution de la solution. Le corps 3 du dispositif de distribution 100 porte la tête 5 pourvue d'une surface d'appui 51 sur une zone du corps d'un patient. La surface d'appui 51 de la tête 5 forme un contour extérieur, et comprends au moins un orifice de distribution de la solution, encore appelée ouverture de distribution.

La tête 5 est couplée au corps 3 par les moyens de liaison 18, 19 dont l'agencement autorise sa rotation par rapport au corps 3 afin de pouvoir modifier la position de la surface d'appui 51 par rapport aux zones d'activation 13 disposées de part et d'autre du corps 3.

Les zone d'activation 13 présentent une surface totale restreinte par rapport à la surface 7 du corps 3 et sont chacune disposées selon un secteur angulaire seulement de la surface extérieure 7 du corps 3, ce qui revient à dire que les zones d'activation 13 présentent une forme distincte d'une forme de révolution.

Le corps 3, la tête 5 et la surface d'appui 51 de la tête 5 présentent un contour dont la forme est distincte de celui d'une forme de révolution.

Le dispositif de distribution 100 peut comprendre des éléments de butée aptes à limiter la rotation relative du corps 3 du dispositif de distribution 100 et de sa tête 5, par exemple selon un angle maximal de 45 degrés ou de 90 degrés par rapport à une position nominale encore appelée position de stockage, utile au conditionnement du dispositif de distribution 100.

De façon optionnelle, les moyens de liaison 18, 19 peuvent comprendre des moyens de rappel de la tête en position de stockage.

Astucieusement la tête 5 est montée détachable par rapport au corps 3, ce qui permet de coupler plusieurs formes de tête sur un même corps 3 de dispositif de distribution 100.

L'invention ne se limite pas aux seuls modes de réalisation décrits ci-avant et d'autres modes de réalisation peuvent être envisagés. Selon une variante, la connexion entre une unité de contrôle embarquée dans le corps et la tête portant des moyens de détection optoélectroniques peut être une connexion sans-fil.

Un autre mode de réalisation peut concerner, par exemple, un dispositif de distribution adapté à l'utilisation de têtes thermo-formables pour accroître le confort d'utilisation. Selon un autre exemple, le dispositif de distribution peut être un dispositif d'assistance à la distribution configuré pour accueillir un réservoir extractible et donc interchangeable d'une solution. Ces exemples d'autres modes de réalisations ne sont pas limitatifs.

## Revendications

1. Dispositif de distribution (100) d'une solution comprenant :
- un corps (3) dont la surface extérieure (7) est destinée à être tenue par au moins une main d'un utilisateur et comprenant au moins une zone d'activation (13) apte à être sollicitée par l'utilisateur pour provoquer une distribution de la solution, et,
- une tête (5) portée par le corps (3), la tête (5) étant pourvue d'une surface d'appui (51) sur une zone du corps d'un patient, formant un contour extérieur, et d'au moins un orifice de distribution (52) de la solution,
le dispositif de distribution (100) étant **caractérisé en ce que** la tête (5) est couplée au corps (3) par des moyens de liaison (18, 19) autorisant sa rotation par rapport au corps (3) autour d'un axe de rotation (9) sensiblement parallèle à un axe du corps (3) afin de modifier la position de la surface d'appui (51) par rapport à la au moins une zone d'activation (13).

2. Dispositif de distribution (100) selon la revendication précédente, dans lequel la au moins une zone d'activation (13) présente une surface restreinte, disposée selon un secteur angulaire seulement de la surface extérieure (7) du corps (3).

3. Dispositif de distribution (100) selon l'une quelconque des revendications précédentes, dans lequel la tête (5) a un contour dont la forme est distincte d'une forme de révolution.

4. Dispositif de distribution (100) selon l'une quelconque des revendications précédentes, dans lequel le corps (3) a un contour dont la forme est distincte d'une forme de révolution.

5. Dispositif de distribution (100) selon l'une quelconque des revendications précédentes, dans lequel la liaison opérée par les moyens de liaison (18, 19) est choisie dans le groupe comprenant au moins : une liaison pivot, une liaison rotule, une liaison pivot glissant.

6. Dispositif de distribution (100) selon l'une quelconque des revendications précédentes, dans lequel les moyens de liaison (18, 19) comprennent des éléments de butée aptes à limiter la rotation relative du corps (3) du dispositif de distribution (100) et de sa tête (5).

7. Dispositif de distribution (100) selon l'une quelconque des revendications précédentes, dans lequel la tête (5) et le corps (3) peuvent prendre une position relative dite de stockage et les moyens de liaison (18, 19) comprennent des moyens de rappel de la tête (5) vers sa position de stockage.

8. Dispositif de distribution (100) selon l'une quelconque des revendications précédentes, dans lequel la tête (5) est montée détachable par rapport au corps (3).

## Patentansprüche

1. Vorrichtung (100) zur Abgabe einer Lösung, aufweisend:
- einen Körper (3), dessen Außenfläche (7) dazu bestimmt ist, von mindestens einer Hand eines Benutzers gehalten zu werden, und der mindestens einen Aktivierungsbereich (13) aufweist, der vom Benutzer beansprucht werden kann, um eine Abgabe der Lösung zu bewirken, und,
- einen Kopf (5), der von dem Körper (3) getragen wird, wobei der Kopf (5) mit einer Auflagefläche (51) auf einen Bereich des Körpers eines Patienten versehen ist, die eine Außenkontur bildet, und mit mindestens einer Abgabeöffnung (52) für die Lösung versehen ist,
wobei die Abgabevorrichtung (100) **dadurch gekennzeichnet ist, dass** der Kopf (5) mit dem Körper (3) durch Verbindungsmittel (18, 19) gekoppelt ist, die seine Drehung in Bezug auf den Körper (3) um eine Drehachse (9) erlauben, die im Wesentlichen parallel zu einer Achse des Körpers (3) verläuft, um die Position der Auflagefläche (51) in Bezug auf den mindestens einen Aktivierungsbereich (13) zu verändern.

2. Abgabevorrichtung (100) nach dem vorhergehenden Anspruch, wobei der mindestens eine Aktivierungsbereich (13) eine begrenzte Fläche aufweist, die nur in einem Winkelsektor der Außenfläche (7) des Körpers (3) angeordnet ist.

3. Abgabevorrichtung (100) nach einem der vorhergehenden Ansprüche, wobei der Kopf (5) eine Kontur aufweist, deren Form von einer Rotationsform verschieden ist.

4. Abgabevorrichtung (100) nach einem der vorhergehenden Ansprüche, wobei der Körper (3) eine Kontur aufweist, deren Form von einer Rotationsform verschieden ist.

5. Abgabevorrichtung (100) nach einem der vorhergehenden Ansprüche, wobei die durch die Verbindungsmittel (18, 19) bewirkte Verbindung aus der Gruppe ausgewählt ist, die mindestens aufweist: eine Drehverbindung, eine Kugelgelenkverbindung, eine Gleitdrehverbindung.

6. Abgabevorrichtung (100) nach einem der vorhergehenden Ansprüche, wobei die Verbindungsmittel (18, 19) Anschlagelemente aufweisen, die geeignet sind, die relative Drehung des Körpers (3) der Abgabevorrichtung (100) und ihres Kopfes (5) zu begrenzen.

7. Abgabevorrichtung (100) nach einem der vorhergehenden Ansprüche, bei der der Kopf (5) und der Körper (3) eine relative Position einnehmen können, die als Lagerposition bezeichnet wird, und die Verbindungsmittel (18, 19) Mittel zum Zurückholen des Kopfes (5) in seine Lagerposition aufweisen.

8. Abgabevorrichtung (100) nach einem der vorhergehenden Ansprüche, wobei der Kopf (5) in Bezug auf den Körper (3) lösbar angebracht ist.

## Claims

1. Dispensing device (100) for dispensing a solution, comprising:
- a body (3), the outer surface (7) of which is intended to be held in at least one of a user's hands, and comprising at least one activation zone (13) that is able to be acted upon by the user to cause the solution to be dispensed, and,
- a head (5) carried by the body (3), the head (5) being provided with a contact surface (51) for pressing on an area of a patient's body, the contact surface (51) forming an outer contour, and with at least one orifice (52) for dispensing the solution,
the dispensing device (100) being **characterised in that** the head (5) is coupled to the body (3) by connecting means (18, 19) that allow it to rotate with respect to the body (3) about an axis of rotation (9) substantially parallel to an axis of the body (3) in order to modify the position of the contact surface (51) with respect to the at least one activation zone (13).

2. Dispensing device (100) according to the preceding claim, wherein the at least one activation zone (13) has a restricted surface, arranged according to only an angular sector of the outer surface (7) of the body (3).

3. Dispensing device (100) according to any one of the preceding claims, wherein the head (5) has a contour whose shape is different from a shape of revolution.

4. Dispensing device (100) according to any one of the preceding claims, wherein the body (3) has a contour whose shape is different from a shape of revolution.

5. Dispensing device (100) according to any one of the preceding claims, wherein the connection carried out by the connecting means (18, 19) is chosen from the group comprising at least: a pivot connection, a ball joint connection, a sliding pivot connection.

6. Dispensing device (100) according to any one of the preceding claims, wherein the connecting means (18, 19) comprise stopper elements which are able to limit the relative rotation of the body (3) of the dispensing device (100) and of its head (5).

7. Dispensing device (100) according to any one of the preceding claims, wherein the head (5) and the body (3) can take a relative position called storage position and the connecting means (18, 19) comprise means for returning the head (5) to its storage position.

8. Dispensing device (100) according to any one of the preceding claims, wherein the head (5) is removably mounted with respect to the body (3).
